# EUROPEAN PATENT APPLICATION

(11) **EP 4 249 022 A1**
(43) Date of publication of application: **27.09.2023**
(21) Application number: 22163319.1
(22) Date of filing: 21.03.2022
(51) Int. Cl.: A61M 15/00, A61K 38/19

(54) **DRUG-DEVICE COMBINATION**

(71) Applicant: PARI Pharma GmbH, 82319 Starnberg (DE); Savara Inc., Austin, TX 78746 (US)
(72) Inventor: HETZER, Uwe, 81476 München (DE); TSERVISTAS, Markus, 81479 München (DE); SEEMANN, Stefan, 82467 Garmisch-Partenkirchen (DE)
(74) Representative: Hoffmann Eitle

(57) **Abstract**

The present disclosure relates to a drug-device combination comprising a liquid solution (26) containing a biologic; and a nebulizer (10) for aerosolization of the liquid solution (26), the nebulizer (10) comprising a reservoir (24) for holding the liquid solution (26), the reservoir (24) having a vent (48) for maintaining atmospheric pressure in the reservoir (24), a vibratable membrane (60) having an active area (62) with apertures (110), wherein the liquid solution (26) is feedable to the active area (62) at a liquid side (64) of the membrane (60), a piezoelectric actuator for vibrating the membrane (60), whereby an aerosol of the liquid solution (26) is generated at the active area (62) at an aerosol side (66) of the membrane (60), the aerosol side (66) of the membrane (60) being opposite to the liquid side (64) of the membrane (60) and a plenum chamber (23) having an inlet (76) at an inlet side at the aerosol side (66) of the membrane (60) and an outlet (94) at an outlet side for administering the aerosol, the outlet (94)of the plenum chamber being opposite to the inlet (76) of the plenum chamber (23).

## Description

### Technical Field

The present disclosure relates to a drug-device combination, particularly a drug-device combination comprising a liquid solution containing a pharmaceutical ingredient and a nebulizer for aerosolization of the liquid solution.

### Background

Nebulization of liquid solutions containing pharmaceutical ingredients (APIs) has many advantages for delivering medications to the lung. However, each pharmaceutical ingredient behaves differently when a liquid solution (carrier fluid) containing the pharmaceutical ingredient is converted into an aerosol by the nebulizer. Different chemical properties inherent to the pharmaceutical ingredient and/or the liquid solution dictate the device and delivery parameters that enable delivery of a therapeutically effective dose of the pharmaceutical ingredient as an aerosol. That is, the selection of a nebulizer device for one pharmaceutical ingredient may not hold for a different pharmaceutical ingredient.

### Summary

In view of the aforesaid it is an aim to provide a nebulizer for aerosolization of a liquid solution containing a biologic enabling delivery of a therapeutically effective dose of the biologic as an aerosol.

In this regard, a biologic drug (biologics) is a product that is produced from living organisms or contain components of living organisms. Biologic drugs include a wide variety of products derived from human, animal, or microorganisms by using biotechnology. Types of biologic drugs include vaccines, blood, blood components, cells, allergens, genes, tissues, and recombinant proteins. Biologic products may contain proteins that control the action of other proteins and cellular processes, genes that control production of vital proteins, modified human hormones, or cells that produce substances that suppress or activate components of the immune system. Biologic drugs are sometimes referred to as biologic response modifiers because they change the manner of operation of natural biologic intracellular and cellular actions.

According to an aspect of the present disclosure, a drug-device combination is suggested comprising a liquid solution containing a biologic and a nebulizer for aerosolization of the liquid solution.

The nebulizer comprises a reservoir for holding the liquid solution and a vibratable membrane having an active area with apertures, wherein the liquid solution is feedable to the active area at a liquid side of the membrane, for example by gravitational force. The nebulizer further comprises a piezoelectric actuator for vibrating the membrane, whereby an aerosol of the liquid solution is generated at the active area at an aerosol side of the membrane, the aerosol side of the membrane being opposite to the liquid side of the membrane. The vibratable membrane may function as a flexural transducer. The piezoelectric actuator may directly act on the vibratable membrane or a substrate to which the vibratable membrane is attached. The piezoelectric actuator may be directly attached to the vibratable membrane or the substrate.

It has been found advantageous for the aerosolization of a liquid solution containing a biologic to use a nebulizer having a vibratable membrane because the shear forces acting on the biologic at the time the aerosol is generated are small and destruction of the biologic can be minimized or even be avoided by use of such membrane nebulizer.

Additionally, the energy is only supplied to the piezoelectric actuator and from the piezoelectric actuator only to the vibratable membrane. Accordingly, only gentle energy input is required for aerosolization of the liquid solution. Due to the gentle energy input no or little heat is generated with the benefit that no or only little heat is transferred to the liquid solution. Consequently, destruction of the biologic may be further minimized or even be avoided.

However, it has been found that, when using a nebulizer having a vibratable membrane vibrated by a piezoelectric actuator, the liquid solution comprising the biologic tends to foam. This phenomenon has been associated by the present inventors to the existence of a negative pressure in the reservoir. Such negative pressure may be generated during the nebulization process due to the decreasing amount of liquid solution in a sealed reservoir or may even be created by purpose already before the nebulization process starts as suggested in e.g. WO 1997/29851 A1 or US 6,983,747 B2 in order to increase the efficiency and the total output rate. Due to such negative pressure air may be sucked through the apertures in the membrane into the reservoir resulting in the generation of foam in the reservoir. The generation of foam increases the surface of the liquid solution and, hence, the biologic. Further, oxygen is introduced into the liquid solution so that the biologic denaturizes. As a consequence, the quality of the aerosol is deteriorated. The formation of foam may even lead to termination of the nebulizing process. To avoid or at least minimize the tendency of the liquid solution within the reservoir to foam, the reservoir has a vent for maintaining atmospheric pressure (atmospheric pressure +/- 10mbar) in the reservoir during the (entire) nebulizing process.

The nebulizer further comprises a plenum chamber having an inlet at an inlet side at the aerosol side of the membrane and an outlet at an outlet side for administering the aerosol, the outlet of the plenum chamber being opposite to the inlet of the plenum chamber. In a particular embodiment, the plenum chamber may be defined between the inlet of a mixing chamber at the aerosol side of the membrane and the outlet of a mouthpiece or a mask covering nose and mouth. In other words, the plenum chamber may be defined by the membrane at one end and the outlet of the mouthpiece/mask at the other end with the mixing chamber and the mouthpiece/mask being positioned in between. The mouthpiece or mask may be coupled to an interface at an outlet side if the mixing chamber. The interface may be as simple as a cylindrical socket.

The use of the plenum chamber in connection with a liquid solution containing a biologic has proven advantageous in order to reduce the amount of residue in the nebulizer due to increased condensation of aerosol. Thus, a relatively high delivered dose of liquid solution may be realized.

The vent may be a passive vent. A passive vent is a cost-effective solution not requiring additional parts. The size/dimension of the passive vent may be selected so as to realize the venting function for avoiding foaming of the liquid solution in the reservoir and at the same time prevent the liquid solution from leaking out of the reservoir through the vent, e.g. when the nebulizer is tilted during use. Thus, the area of the passive vent may be between 0,5mm² and 5mm² or between 0,5mm² and 2mm². In an embodiment, the area of the passive vent may be less than 5mm², less 4mm², less than 3mm² or less than 2mm². In an example the area of the passive vent may be between 0,8mm² and 1mm². Its width may be between 0,5mm and 3mm or between 0,5mm and 2mm. The height may be between 0,5mm and 3mm or between 0,5mm and 2mm. Its depth may be more than 1mm, more than 2mm, more than 2,5mm. The depth may be between 1mm and 4mm or between 2mm and 3mm. Using those dimensions avoid leaking of the liquid solution.

The reservoir may be an ampoule or a vessel to be opened within the nebulizer to feed the liquid solution to the liquid side of the active area of the membrane, wherein the vent is either formed before inserting the ampoule or when inserting the ampoule into the nebulizer, e.g. by piercing the ampoule. Yet, according to an aspect of the present disclosure, the reservoir is a cup reservoir having a body (housing) sealed at a lower portion thereof by the liquid side of the vibratable membrane, particularly its active area or an area surrounding the active area. The body has a circumferential rim defining an opening for receiving the liquid solution and a sealing surface. The liquid solution may be poured into the reservoir via the opening. Further, a lid (closure) is provided for engaging the circumferential rim to close the reservoir and sealing against the sealing surface. The vent is formed so as to connect a head space of the reservoir to atmosphere and spaced away from the lower portion sealed by the vibratable membrane. The head space is the portion in the reservoir above the liquid solution and filled with air. In particular. The vent may be provided in the vent may be provided in the circumferential rim. In this case, the depth of the vent is governed by the width of the circumferential rim. Consequently, the vent may be realized in a simple and effective manner, on the one hand, providing effective venting of the reservoir to avoid the mentioned foaming and, on the other hand, preventing liquid solution from leaking out of the reservoir through the vent.

The vent may be a notch formed from the sealing surface into the circumferential rim and traversing the circumferential rim connecting the interior of the reservoir and atmosphere.

The provision of the vent as a notch in the circumferential rim of the reservoir provides for an easy solution. Additionally, a notch is open at the sealing surface and therefore simplifies the cleaning process. Hence, this configuration is beneficial from a hygienic aspect.

The plenum chamber may have a volume of at least 60 cubic centimeters and/or not more than 90 cubic centimeters. The plenum chamber may have a length between 104mm and 154mm and/or a width of 40mm and 60mm and/or a height of 30mm and 50mm. The mixing chamber of the plenum chamber as such may have a length between 54mm and 94mm and/or a width of 40mm and 60mm and/or a height of 30mm and 50mm. The height of the plenum chamber and/or the mixing chamber are factors to minimize sedimentation of the aerosol in the plenum chamber/mixing chamber. The length serves calming the air flow during inhalation with the aim to come as close to a laminar flow as possible.

The apertures of the vibratable membrane may have a size at the aerosol side between 1,0 µm to 5 µm or between 1,5 µm to 4,5 µm or between 2,0 µm and 4,0 µm.

The vibratable membrane may have 500 to 6000 or 2000 to 6000 or 2800 to 6000 of the apertures. The active area of the vibratable membrane may have a diameter of 3mm to 6mm and the distance between the apertures in the active area may be between 40µm and 90µm or between 40µm and 80µm, such as 70µm. In one example the active area of the vibratable membrane may have a diameter of 4mm and the distance between the apertures in the active area may be 70µm resulting in around 3.000 apertures in the active area.

The apertures may be laser drilled and/or laser milled. According to one aspect, the through holes may be laser-drilled through holes formed in at least two stages, one stage forming a nozzle portion and the remaining stage/-s forming the remainder of the through holes. In another aspect, the vibratable membrane comprises a single (one single) layer of unitary material. A single layer of unitary material has no distinct borders, boundary surfaces or confining layers or the like in a polished cut image of the single layer. The vibratable membrane may in one embodiment comprise further layers attached to the single layer. In other embodiments, the vibratable membrane may consist of the single layer. A plurality of recesses may be formed in the single layer. The recesses each have an opening facing the fluid side or - in case the vibratable membrane consists of the single layer - at the fluid side of the vibratable membrane. The recesses each have a bottom opposite to the opening, i.e. at a side facing the aerosol side or at the aerosol side of the vibratable membrane. The recesses may also be referred to as blind holes introduced from one side of the single layer (from the fluid side (first side) of the vibratable membrane). In one aspect, at least two recesses are provided. In another aspect, at least three recesses are provided. Further, the number of recesses is not limited but less than 3,000, less than 1,000 or less than 500 may be preferred. Alternatively, less than 50 or less than 20 recesses may be provided. A relatively large number of smaller recesses, such as between 500 and 3,000 or 500 and 1,000, may be beneficial to achieve a high stability of the vibratable membrane. The more recesses are provided in the vibratable membrane, the higher the remaining reinforcing portions between the recesses providing for the full thickness of the single material layer and, hence, stability. To the contrary, a smaller number of larger recesses, such as between 2 and 50 or 2 and 20, may be beneficial to achieve a larger effective area for nebulization with less dead areas. In this context, "dead areas" are those areas of the vibratable membrane having no penetrating apertures and, hence, do not participate in the nebulization process. To put it differently, in these areas no fluid may pass through the vibratable membrane for being nebulized at the fluid side. Additionally, the manufacturing process may be simplified when using a smaller number of recesses. The area of the recesses relative to the total area of the vibratable membrane, in one example, not exceed 50%. Further, a plurality of apertures is formed in the bottom of each of the recesses. The plurality of apertures may each have an entrance opening formed in the bottom of the respective recess and an exit opening facing or formed at the aerosol side of the vibratable membrane. According to an aspect, there are no additional apertures formed in the vibratable membrane other than those formed in the bottom of the recesses. According to another aspect, there are at least no additional apertures formed in the single layer between recesses and being blind holes. Thus, the fluid on the fluid side of the vibratable membrane passes, upon vibration of the vibratable membrane, through the recesses and apertures and is nebulized while exiting the apertures via the exit openings at the aerosol side of the vibratable membrane. Thus, an aerosol is formed at the aerosol side of the vibratable membrane for inhalation by a patient. In this aspect, the entrance opening of the apertures can be relatively small so that the density of the apertures may be increased as compared to the density of the three-stage laser-drilled apertures, thus enabling to increase the TOR. Even further, the TOR and MMD are strongly dependent on the geometry of the nozzle portion, i.e. of the aperture geometry (particularly length and diameter). Due to the formation of the recesses, a small and relatively even or constant thickness of the bottom of the recesses is achieved. Accordingly, the length of the apertures is very well defined. In addition, the formation of relatively short apertures is much more precise regarding diameter at an entrance side (corresponding to the fluid side) and an exit side (corresponding to the aerosol side). As a result, a much more consistent geometry of the apertures relative to each other may be achieved, reducing the GSD. Hence, the characteristics of the aerosol (nebulized fluid) are more consistent when comparing different aperture plates obtained from the same manufacturing process. In one embodiment, however, the recesses are laser milled and/or the apertures are laser drilled. The use of a laser milling process for forming the recesses is also beneficial from the viewpoint of achieving a relatively even and constant remaining material thickness in the area of the bottom of the recesses. Thus, an even more precise formation of the apertures regarding diameter and length may be achieved with the above described benefits relating to reproducibility of constant aerosol characteristics. In a specific example, an ultra-short-pulse laser is used for laser milling. An ultra-short-pulse laser is defined as a laser using laser pulses of less than 10 picoseconds. An ultra-short-pulse laser has the benefit of being more precise and even further improving the benefits relating to reproducibility of constant aerosol characteristics described above. The apertures may still be formed by using a short-pulse laser (e.g. a nanosecond laser) or by using an ultra-short-pulse laser. A short-pulse laser is defined as a laser using laser pulses of greater than 10 picoseconds and smaller than 500 nanoseconds.

The apertures may have a (the) nozzle portion at the aerosol side of the active area of the membrane, the nozzle portion having a length between 10 µm and 25 µm or 10 µm and 20 µm or 10 µm and 15 µm. The length may be less than 20µm or less than 18µm or less than 16µm. The diameter of the nozzle portion at the aerosol side may be between 1,5 µm to 5 µm or between 1,5 µm to 3 µm. One end of the nozzle portion terminates flush with the aerosol side of the vibratable membrane. The diameter may be measured at the one end and should reside within the above range. Such a defined nozzle portion reduces the shear forces acting on the liquid solution and may, as explained above, avoid or minimize destruction of the biological contained therein.

The liquid solution may have a viscosity of between 0.900 mPa•s to 5.000 mPa•s or preferred between 0.950 mPa•s and 3.500 mPa•s, or more preferred between 1.000 mPa•s and 2.000 mPa•s, or even more preferred between 1.000 mPa•s and 1.800 mPa•s or most preferred between 1.000 mPa•s and 1.400 mPa•s.

The liquid solution may have a surface tension of 32 mN/m to 108 mN/m, or preferred 50 mN/m to 100 mN/m, or more preferred 52 mN/m to 78 mN/m, or even more preferred of 57 mN/m to 73 mN/m, or most preferred from 58 mN/m to 68 mN/m. In another case the liquid solution may have a surface tension of 32 mN/m to 108 mN/m, or preferred 32 mN/m to 78 mN/m, or preferred of 37 mN/m to 73 mN/m, or more preferred from 58 mN/m to 68 mN/m.

The liquid solution may have an osmolality of 200 mOsmo/kg to 700 mOsmo/kg, preferred 200 mOsmo/kg to 500 mOsmo/kg, or even more preferred 300 mOsmo/kg to 350 mOsmo/kg.

The amount of the liquid solution of one dose held in the reservoir is between 1 ml and 2 ml.

The volume of the reservoir may be between 1 ml and 8 ml or between 3 ml and 5 ml.

The biologic may be from the group of major kinds of biopharmaceuticals include Blood factors (Factor VIII and Factor IX), Thrombolytic agents (tissue plasminogen activator), Hormones (insulin, glucagon, growth hormone, gonadotrophins), Haematopoietic growth factors (Erythropoietin, colony-stimulating factors), Interferons (Interferons-a, -β, -γ), Interleukin-based products (Interleukin-2), Vaccines (Hepatitis B surface antigen) Monoclonal antibodies (Various), Polyclonal antibodies, Proteins, Peptides, tRNA, siRNA, mRNA, RNA interference, Antisense oligonucleotides, Surfactant, immunoglobine (IgG, IgM, IgAM), Bacteriophages, Exosomes, Stem cells, Cells, Recombinant viruses, Bacteria, Enzymes Hormones, Insulin, Additional products (tumour necrosis factor, therapeutic enzymes), and similar biopharmaceuticals or similar drug products.

The biologic may comprise one or more proteins. Potential proteins are for example immunoglobin (A, G, M, ...), immunoglobin CTLA-4 fusion protein, monoclonal antibody, Polyclonal antibodies, immunoglobin G1 fusion protein, recombinant protein, recombinant human TNF-receptor fusion protein, monoclonal antibody, humanized monoclonal antibody, Diphtheria toxin engineered protein combining Interleukin-2 and Diphtheria toxin, and similar proteins or similar protein fragments.

The protein may in a specific aspect be granulocyte-macrophage colony stimulating factor (GM-CSF) and in a more specific aspect be recombinant human granulocyte-macrophage colony stimulating factor (rhGM-CSF).

The biologic may comprise at least two or more proteins. Therefor at least a second protein may be added, most useful a carrier, an excipient and/or the like, for example Human Serum Albumin (HA) or especially recombinant Human Serum Albumin (rHA).

### Brief Description of the Drawings

Figure 1 shows a perspective view of a nebulizer of an embodiment of the drug device combination.
Figure 2 shows an explosive view of the nebulizer.
Figure 3A and 3B are longitudinal cross sections of the nebulizer of an embodiment without ribs in a state (A) in which the lid is only placed on the body and a state (B) in which the lid is screwed and fixed to the body and Figure 3 (C) is an enlarged partial longitudinal cross sections of the nebulizer of an embodiment with ribs in a state in which the lid is only placed on the body.
Figure 4 is a longitudinal cross section of only the body defining the reservoir.
Figure 5 is a perspective view of the body of the nebulizer defining the reservoir.
Figure 6 as an enlarged portion of the body of the nebulizer shown in figure 5.
Figure 7 shows a partial cross section of the active area of the vibratable membrane according to an embodiment.
Figure 8 shows a partial cross section of the active area of the vibratable membrane according to another embodiment.

### Detailed Description

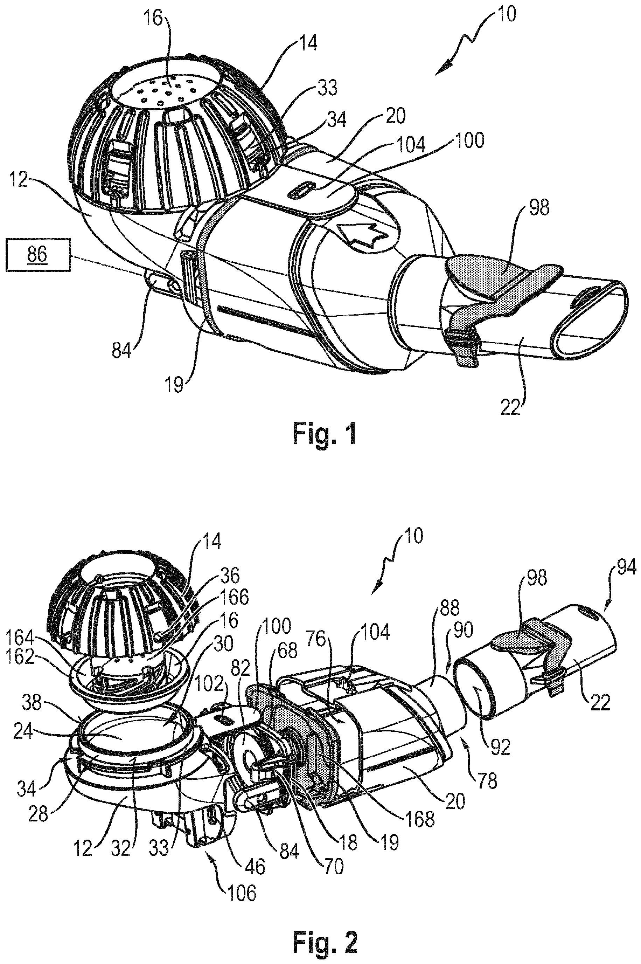

The nebulizer 10 shown in figures 1 to 3 comprises a body 12 (housing), a lid (closure) 14, a sealing 16, an aerosol generator 18, an elastic valve member 19, a mixing chamber 20 and a mouthpiece 22.

The body 12 defines a reservoir 24 for holding a liquid solution 26 containing a biologic. The biologic may comprise a protein. The protein may be recombinant human granulocyte-macrophage colony stimulating factor (rhGM-CSF). In a particular embodiment, the human GM-CSF may be manufactured in E.coli as compared to other recombinant human GM-CSF proteins manufactured in other expression systems (e.g. yeast or mammalian cells). Human GM-CSF has a molecular weight of ∼28 to 32kDa, whereas the recombinant human GM-CSF (manufactured in E.coli) of an embodiment may have a molecular weight of 14.5 kDa. The difference is due to the absence of glycosylation on the molecule of the present recombinant human GM-CSF. Hence, the SVRA protein may be called: "molgramostim, a recombinant human GM-CSF manufactured in bacterial cells (hereafter termed GM-CSF)".

The liquid solution may have a viscosity of 1.000 to 1.400 mPa•s. The liquid solution may have a surface tension of 37 mN/m to 73 mN/m. The liquid solution may have an osmolality of 300 to 350 mOsm/kg.

In a specific example, the composition was as shown in Table 1 below.

**Tab. 1: Composition**

| **Component** | **Concentration in aqueous formulation [.../mL]** | **Function** |
|---|---|---|
| rhGM-CSF | 250 µg | API |
| Mannitol | 50 mg | Exipient |
| PEG 4000 | 100 µg | Surfactatnt |
| rHA (recombinant Human Serum Albumin) | 1 mg | Carrier |
| Citric acid (mono hydrate) | 0.28 mg | Buffer component |
| Na₂HPO₄ | 2.6 mg | Buffer component |

In a specific example, the physicochemical parameter of 250µg/mL formulation were as shown in Table 2.

**Tab. 2: Physicochemical Parameters of 250 µg/mL rhGM-CSF Formulation**

| Parameter | Value |
|---|---|
| Density | 1.054 g/mL |
| Dynamic Viscosity (20°C) | 1.19 mPa·s |
| Surface tension (20°C) | 63.6 mN/m |

The density and viscosity are slightly increased, whereas surface tension is reduced compared to pure water.

The amount of the liquid solution of one dose may be between 1 ml and 2 ml. In this context, one dose is to be understood as one dose of an administration strategy and to be administered at once, e.g. without stopping the nebulizer 10, i.e. vibration of the membrane 61 and aerosol production, or re-opening the lid 14.

The reservoir 24 may have a volume between 1 ml and 8 ml, preferably between 3ml and 5ml. In the present embodiment, the reservoir 24 is a cup shaped reservoir. The body 12 has a circumferential rim 28 at its upper portion defining an opening 30 communicating with the reservoir 24. The area of opening 30 may be substantially (± 20°) horizontally oriented when the nebulizer is placed on a horizontal surface. The liquid solution 26 may be poured into the reservoir 24 via the opening 30. For example, the liquid solution may be held in an ampoule and may be poured into the reservoir 24 upon opening of the ampoule.

An outer circumferential surface 32 of the rim 28 may be provided with a helical path 33 having a plurality of slots 34 as part of a bayonet closure/mount for attaching the lid 14 to the body 12. The lid 14 has, as counter parts to the slots 34, protrusions 36 (lugs) protruding radially inwardly from an inner surface of the lid 14. When attaching the lid 14 to the body 12, the protrusions 36 pass the helical path 33 by entering the slots 34. Upon rotation of the lid 14, the lid 14 is locked to the body 12 in that the protrusions 36 are caught behind the helical path 33.

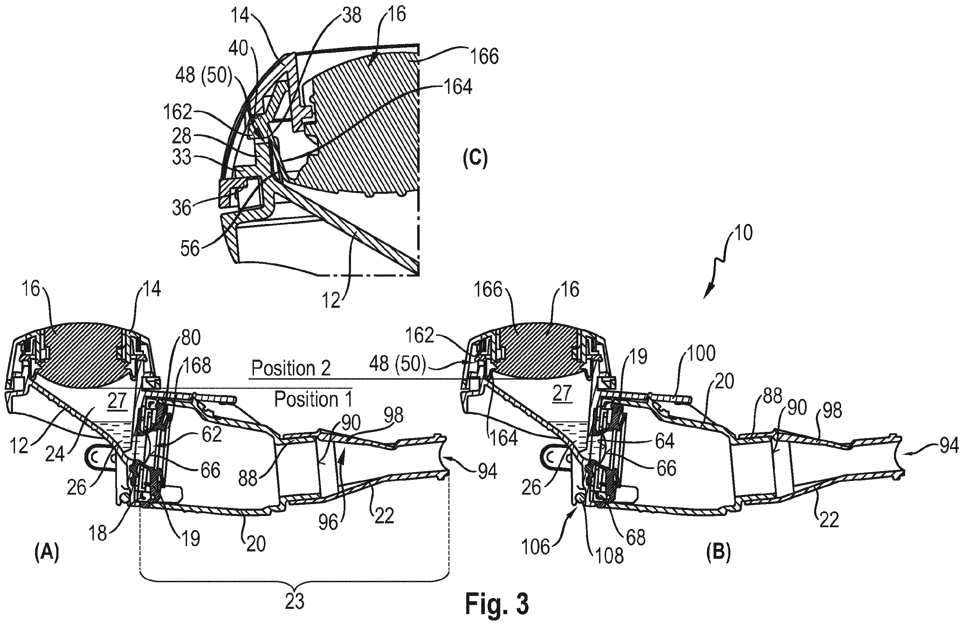

A top surface (sealing surface) 38 of the rim 28 serves as a first counter surface cooperating with the sealing 16 for a sealing the opening 30 upon attachment of the lid 14 onto the body 12. For this purpose, the lid 14 has a corresponding second counter surface 40 (see figure 3C). When attaching the lid 14 to the body 12 and rotating the lid 14, the lid 14 is, due to the helical path 33 pushed towards the body. Accordingly, the second counter surface 40 of the lid 14 is pushed towards the top surface 38 of the rim 28 and, hence, the sealing 16 is sandwiched/clamped between the top surface 38 and the second counter surface 40, whereby the opening 30 is sealed.

The sealing 16 may comprise a sealing ring 162 as a portion which is sandwiched/clamped between the top surface 38 and the second counter surface 40. The sealing 16 may further comprise a movable sealing hinge 164 extending from the sealing ring 162. The movable sealing hinge 164 may further be connected to a center sealing 166. The center sealing 166 may be held in the lid 14 to be axially movable upon attachment of the lid 14 to the body 12, particularly upon rotation of the lid 14 relative to the body 12 as explained above.

As will be apparent from figures 3a and 3b, the sealing hinge 164 additionally seals against the inner circumferential surface 52 of the rim 28.

Additionally, the center sealing 166 moves from the position 1 in figure 3 (A) to the position 2 in figure 3 (B) upon attachment of the lid 14 to the body 12, wherein a negative pressure may be created in the reservoir 24 as for example described as beneficial in EP 1 353 759 A2. As will be apparent from figure 3, the sealing 16 and the lid 14 are threadedly engaged. As the sealing 16 cannot rotate together with the lid once the sealing ring 164 is sandwiched between the top surface 38 and the counter surface 40, the rotating of the lid 14 is transferred into a translational movement from the position 1 in figure 3 (A) to the position 2 in figure 3 (B).

In order to maintain atmospheric pressure within the reservoir 24 while attaching the lid 14 to the body 12 and/or during nebulization, the reservoir 24 has a vent 48 (here a passive vent). The vent 48 is, in this embodiment, configured as a notch or groove 50 formed from the top surface 38 of the circumferential rim 28. The notch 50 traverses the circumferential rim 28 from the outer circumferential surface 32 to the inner circumferential surface 52 communicating the interior of the reservoir 24 with the outside of the nebulizer 10, i.e. atmosphere. Hence, the notch 50 is open at the top surface 38 and extends from the inner circumferential surface 32 to the outer circumferential surface 52 of the rim 28. The notch 50 has a bottom 54 spaced from the top surface 38. The height of the notch 50 from the bottom 54 to the top surface 38 may be between 0,5mm and 3mm or between 0,5mm and 2mm. A width of the notch 50 in a circumferential direction may be between 0,5mm and 3mm or between 0,5mm and 2mm. As a result, an opening area of the notch 50 on an inner circumferential side 52 of the rim 28 may be between 0,5mm² and 5mm² or between 0,5mm² and 2mm². In an embodiment, the area of the passive vent may be less than 5mm², less 4mm², less than 3mm² or less than 2mm². In an example the area of the passive vent may be between 0,8mm² and 1mm².

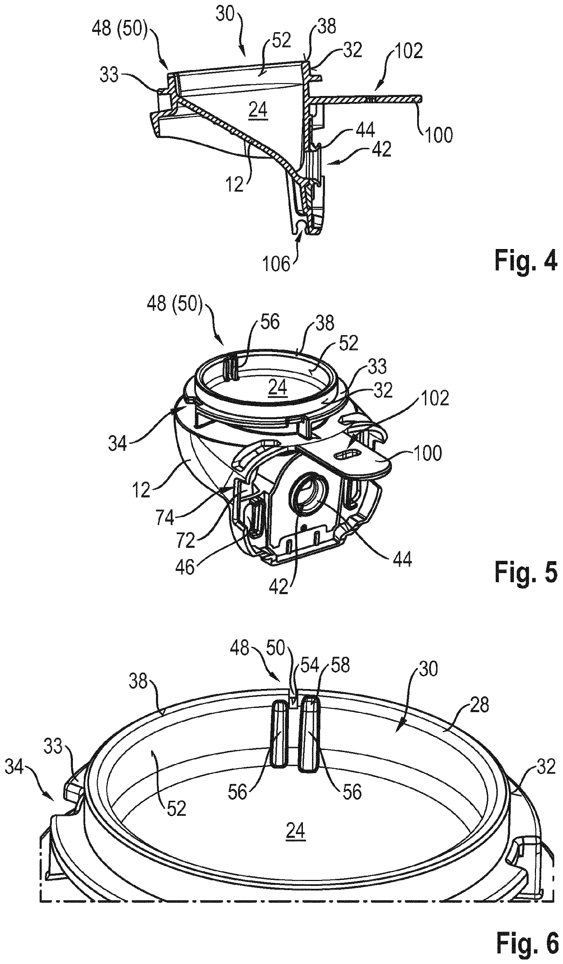

As will be apparent, the vent 48 is provided at the very top of the reservoir 24 and hence in the headspace 27 (see figure 3) of the reservoir 24 above the level of the liquid solution 26 and spaced away from a supply opening 42 (see figures 4 and 5) and, hence, the lower portion of the body 12.

The vent 48 may be provided at the rear side of the body 12 or the nebulizer 10. In an embodiment, the vent 48 may be positioned in a portion of the body 12 opposite to the supply opening 42. Further, the vent 48 may be positioned on the longitudinal centerline of the nebulizer or substantially (within an angle of ±10°) on the longitudinal centerline of the nebulizer.

In order to avoid that the sealing hinge 164 blocks the vent 48 and, therefore, maintain ventilation of the reservoir 24, at least one longitudinal rib 56 may be provided. It is apparent from the enlarged view in figure 3 (C) that the sealing hinge 164 is distanced from the inner circumferential wall 52 by means of the ribs 56 improving ventilation, whereas it is in contact with the inner circumferential wall 52 in figure 3 (A) and (B).

The longitudinal rib 56 may extend parallel to the inner circumferential surface 52 of the rim 28 and protrude radially inwardly. The longitudinal rib 56 may be formed so as to not extend to the top surface 38 of the rim 28. In other words, a top surface 58 of the longitudinal rib 56 is spaced from the top surface 38 of the rim 28. In an embodiment, two of the longitudinal ribs 56 may be provided on circumferentially opposite sides of the vent 48. The ribs 56 may be provided parallel to each other. Further, one of the ribs may be longer in the direction parallel to the inner circumferential surface 52 of the rim 28 than the other one of the ribs 56. Further, the top surface 58 of one of the ribs 56 may be a spaced further away from the top surface 38 of the rim 28 then the top surface 58 of the other one of the ribs 56. Moreover, one of the ribs 56 may protrude further from the inner circumferential surface 52 of the rim 28 than the other one of the ribs 56. In the particular embodiment, the longer rib 56 protrudes further from the inner circumferential surface 52 of the rim 28 than the shorter rib 56. The rib/-s 56 further facilitate removal of the lid 14 preventing the sealing hinge 164 from firmly sticking to the inner circumferential surface 52 (see Figures 3A and 3B).

A lower portion of the body 12 may define a supply opening 42 communicating with the reservoir 24 (see figures 4 and 5). A sealing lip 44 may be provided to the body 12 surrounding the supply opening 42 at the side of the body 12 opposite to the reservoir 24.

The body 12 further has contact openings 46. The contact openings 46 may be formed diametral opposite to the supply opening 42 and the sealing lip 44 and configured to accommodate contacts 84 (see figure 2).

The aerosol generator 18 comprises an aerosol generator 18. The aerosol generator 18 comprises a vibratable membrane 60. The vibratable membrane 60 has an active area 62. The active area 62 may be domed as shown in figures 3a and 3b. Further, the active area 62 comprises a plurality of apertures 110 as shown in figure 6. The vibratable membrane 60 may have 500 to 6000 or 2000 to 6000 or 2800 to 6000 of the apertures. The active area 62 of the vibratable membrane 60 may have a diameter of 3mm to 6mm and the distance between the apertures 110 in the active area 62 may be between 40µm and 90µm or between 40µm and 80µm, such as 70µm. In one example the active area 62 of the vibratable membrane 60 may have a diameter of 4mm and the distance between the apertures 110 in the active area 62 may be 70µm resulting in around 3000 apertures 110 in the active area 62.

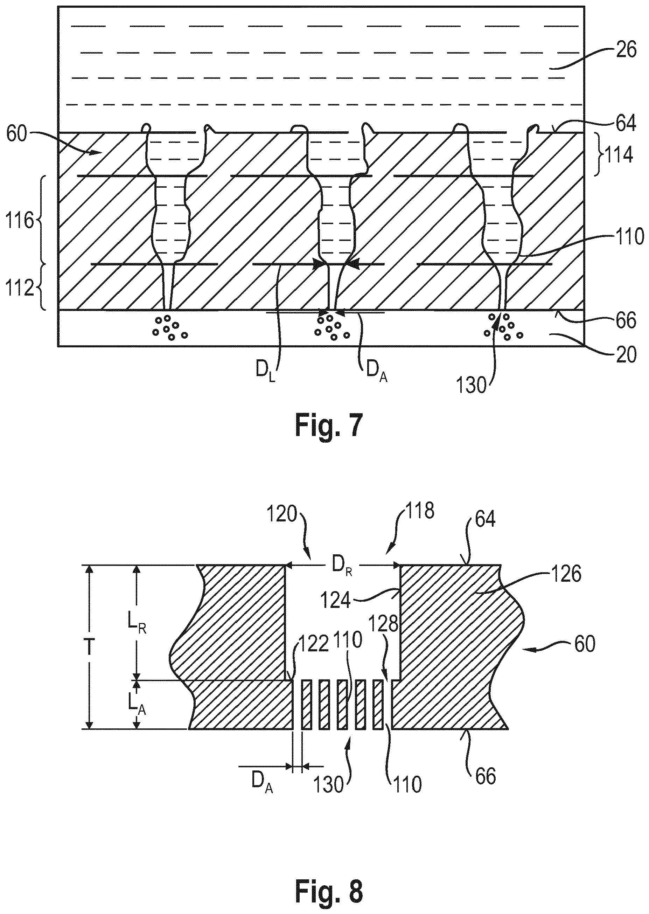

The apertures 110 may laser drilled as shown in Figure 7. The apertures 110 penetrate the membrane 61 in the active area 62 from the liquid side 64 to the aerosol side 66. In use, the liquid solution 26 passes through the apertures 110 from the liquid side 64 to the aerosol side 66 when the membrane 61 is vibrated for generating the aerosol at the aerosol side 66 and emitting it into the mixing chamber 20. This aerosol may then be drawn by inhalation of a patient from the mixing chamber 20 via the mouthpiece 21.

Figure 7 shows a cross-section (schematic CT picture) showing three of the apertures 110 of such a vibratable membrane 60. The through holes 110 of this particular embodiment are formed by laser drilling using three stages of different process parameters, respectively. In a first stage, the portion 114 is formed. In a second stage the portion 116 is formed and in a third stage the nozzle portion 112 is formed. In this embodiment, the average length of the nozzle portion 112 is 26 µm, whereas the second portion 116 has an average length of 51 µm. The first portion 114 has an average length of 24.5 µm. As a result, the total length of each aperture 110 is the sum of the length of the first portion 114, the second portion 116 and the nozzle portion 112, that is in this particular example 101.5 µm. Thus, the ratio of the total length of each aperture 110 to the length of a respective one of the nozzle portions 112 is approximately 3.9.

Alternatively, the apertures 110 may be laser milled and laser drilled as shown in Figure 8.

The vibratable membrane 60 in this example consists of a single layer 126 of stainless steel. Yet, in other embodiments, the vibratable membrane 60 may comprise further layers attached to the liquid side 64 and/or the aerosol side 66. Additionally, other biocompatible metals may be used instead of stainless steel.

The exemplary vibratable membrane 60 comprises a plurality of recesses 118. The recesses 118 may be annular.

The recesses 118 have an opening 120 at the liquid side 64 of the vibratable membrane 60. The recesses 118 are formed as blind holes having a bottom 122 opposite to the opening 120.

The recesses 118 have a circumferential side wall 124. In case of the circular recess 118, the circumferential side wall 124 corresponds to the sheath of a cylinder. Yet, in the cross-section in Figures 8, portions of the circumferential side wall 124 are opposite to (face) each other.

The recesses 118 may be formed in the single layer 128 by using an ultra-short-pulse laser. In this context, the recesses 118 are preferably made by laser milling, wherein the laser beam and/or the single layer 128 of the vibratable membrane 60 are translated relative to each other whereby material of the single layer 128 is gradually removed.

The smallest dimension D_{R} of the recesses 118 may be 300 µm. However, other dimensions are as well conceivable. For example, a smallest dimension D_{R} of the opening 120 of the recesses 118 facing the liquid side 64 may be between 40 µm and 500 µm, between 70 µm and 400 µm or between 90 µm and 300 µm.

In this context, the smallest dimension D_{R} for the circular central recess 118 corresponds to the diameter of the circle. The thickness T of the single layer 128 may be 100 µm.

The depth or length L_{R} of the recesses 118 may be selected between 80% to 95% of the thickness T of the single layer 128. Hence, the depth L_{R} of the recesses 10 may be between 80 µm to 95 µm.

However, the thickness T of the single layer 128 may reside in a range between 50µm to 200µm. Additionally, the depth L_{R} of the recesses 10 may be selected to be equal to or more than 50% of the thickness T of the single layer 128.

Each recess 118 has a plurality of apertures 110 formed in the bottom 122 and extending from the bottom 122 to the aerosol side 66 of the single layer 128.

Each recess 118 may have at least 2, at least 20, or at least 50 or at least 100 of the apertures 110 formed in the bottom 122. At most each recess 118 may have 5,000 or 10,000of the apertures 110.

Each aperture 110 has an entrance opening 128 at the bottom 122 and an exit opening 130 at the aerosol side 66 of the single layer 128. The apertures 110 may be substantially cylindrical or conical with the smaller opening being located at the aerosol side 9 and thus being the exit opening 130. Yet, the geometry is not limited in this regard and other geometries are as well conceivable.

The size of the exit openings 130 of the apertures 110 has a significant impact on the MMD. Depending on the fluid, the MMD is linearly larger than the diameter D_{A} of the exit openings 130 and, thus, directly proportional. Accordingly, the apertures 110 may be circular having a diameter D_{A} (diameter of the exit openings 130) facing the aerosol side 66 between 1 µm and 7 µm, preferably between 1.5 µm and 5 µm, and more preferred between 1.5 µm and 4.5 µm and most preferred between 1.5 µm and 3.5 µm.

A length L_{A} (which may also be referred to as height or depth) of the apertures 110 in the single layer 128 may be between 3 µm and 50 µm. In another example, the length L_{A} of the apertures 118 in the single layer 128 may be between 5 µm and 20 µm. In an even further example, the length L_{A} of the apertures 110 in the single layer 128 may be between 10 µm and 20 µm. Other ranges may be 5 to 15µm, 5 to 12µm or 10 to 15µm.

Even further, the TOR and MMD are strongly dependent on the geometry of the nozzle portion 112, i.e. of the aperture 110 geometry (particularly length and diameter).

The apertures 110 may be formed by the same ultra-short-pulse laser used for forming the recesses 118. However, also a short-pulse laser, as used in the prior art, may be used for forming the apertures 110.

In any case, formation of the apertures 110 is preferably performed in one laser drilling stage at a substantially constant fluence of the laser. Only one drilling stage for forming the apertures is preferred to increase preciseness of the geometry of the apertures, i.e. the nozzle portion 112. When using just one laser drilling stage, the diameter at an entrance side (corresponding to the liquid side) and an exit side (corresponding to the aerosol side) will be very well defined with the above described benefits.

In another aspect, the apertures 110 in the bottom of the recesses 118 may also be drilled with two laser drilling stages having a different fluence similar to the embodiment above using the three drilling stages. For example a first laser drilling stage with a first fluence may be used to form a first part of the aperture in the bottom of the recess and a second laser drilling stage with a second fluence lower than the first fluence may be used to finalize the aperture, i.e. completely penetrate the bottom. In this instance, the nozzle portion may be defined by the second laser drilling stage only. Thus, the bottom may remain thicker (lower depth of the recess) when using more than one laser drilling stage for forming the apertures. This may provide for more mechanical rigidity of the aperture plate.

The method of manufacturing may include as a first step the formation of the recesses 118 from the liquid side 64 of the single layer 128.

In a second, subsequent step, the apertures 110 are formed in the bottoms 122 of the recesses 118 from the liquid side 64 of the single layer 128.

Subsequently, the vibratable membrane 60 may be domed.

The apertures 110 may have a nozzle portion 112 at the aerosol side 66 of the active area 62 of the membrane 61. The nozzle portion 112 may have a length between 10 µm and 25 µm or 10 µm and 20 µm or 10 µm and 15 µm. The length may be less than 20µm or less than 18µm or less than 16µm. The diameter D_{A} of the nozzle portion at the aerosol side 66 may be between 1,5 µm to 5 µm, between 1,5 µm to 4 µm, or between 1,5 µm to 3 µm.

The aerosol generator 18 may further comprise a support 80 and the membrane 61 may be attached to the support 80 (see figure 3. Alternatively, the membrane 61 and the support 80 may be formed as a one piece.

The aerosol generator 18 further comprises a piezoelectric actuator 82 for vibrating the membrane 61 (see figure 2). The piezoelectric actuator 82 may be annular. The piezoelectric actuator 82 may concentrically surround the active area 62 of the membrane 61. The piezoelectric actuator 82 may be attached to the support 80 or, if the support 80 and the membrane 61 are integrally formed, to the membrane 60.

Electrical contacts 84 may be electrically contacted with the piezoelectric actuator 82 to supply an alternating voltage to the piezoelectric actuator 82. The electrical contacts 84 may extend perpendicular from the piezoelectric actuator 82 towards the body 12. When the aerosol generator 18 is attached to the body 12, the electrical contacts 84 project through the contacting openings 46 in the body 12 for being contacted with a controller 86 (schematically shown in figure 1). The controller 86 is configured to control the nebulizer 10. Accordingly, when applying the alternating voltage to the piezoelectric actuator 82 its change in length is directly transferred to the support 80 and from the support 80 to the membrane 61 or even directly to the membrane 61 in case the piezoelectric actuator 82 is directly attached to the membrane 61. Accordingly, the piezoelectric actuator 82 causes the membrane 61 to vibrate such that aerosol is generated at an aerosol generation rate (AGR) in the range of about 0.2 ml/min to about 1.5 ml/min, preferably about 0.4 ml/min to about 1.2 ml/min.

In an embodiment, the aerosol generator 18 is adapted to produce an aerosol fulfilling at least one of the following parameters:
- MMAD ranges from about 1 µm to about 5 µm and is preferably less than 4.5µm or less than 4.0 µm
- MMAD ranges from about 3.0 to about 3.6 µm
- Fine Particle Dose (FPD, <5µm) is greater than 60% of emitted dose or greater than 70% of emitted dose
- Delivered Dose under simulated breathing is greater than 45% or greater than 50%
- TOR (Total Output Rate) more than 0.45 g/min or even more than 0.5 g/min

In a specific example the above defined composition was nebulized. In Tables 3 and 4 all data from laser diffraction measurements of the nebulizers with a plenum chamber 32 (described in more detail below) of more than 50 ml volume and a vented reservoir combined with aerosol generators 18 as defined as a Type MS-3 aerosol generator with a MMAD from 2.8 µm to 3.6 µm and as a Type MS-4 aerosol generator with a MMAD from 3.8 µm to 4.6 µm are summarized below.

**Tab. 3: Data of Laser diffraction measurement with a modified eFlow nebulizer MS-3 (vented reservoir)**

| **Type MS-3 aerosol generator** | **MMD** | **GSD** | **RF < 5 µm** | **RF < 3.3 µm** | **TOR** |
|---|---|---|---|---|---|
| | **[µm]** | | **[%]** | **[%]** | **[mg/min]** |
| MS-3-0554 | 3.26 | 1.55 | 82.70 | 51.12 | 566 |
| | 3.32 | 1.57 | 81.08 | 49.55 | 529 |
| MS-3-0595 | 3.36 | 1.58 | 79.56 | 48.53 | 690 |
| | 3.36 | 1.58 | 79.53 | 48.43 | 701 |
| **Mean** | **3.33** | **1.57** | **80.72** | **49.41** | **621** |
| **SD** | 0.05 | 0.01 | 1.51 | 1.25 | 86 |
| **RSD [%]** | 1.4 | 0.9 | 1.9 | 2.5 | 14.0 |

**Tab. 4: Data of Laser diffraction measurement with modified eFlow nebulizer MS-4 (vented reservoir)**

| **Type MS-4 aerosol generator** | **MMD** | **GSD** | **RF < 5 µm** | **RF < 3.3 µm** | **TOR** |
|---|---|---|---|---|---|
| | **[µm]** | | **[%]** | **[%]** | **[mg/ml]** |
| MS-4-9982 | 4.20 | 1.78 | 61.74 | 33.70 | 1271 |
| | 4.24 | 1.78 | 61.09 | 33.17 | 1291 |
| MS-4-09613 | 4.15 | 1.75 | 62.79 | 34.12 | 1041 |
| | 3.97 | 1.68 | 66.91 | 36.25 | 1001 |
| **Mean** | 4.14 | 1.75 | 63.13 | 34.31 | 1151 |
| **SD** | 0.12 | 0.05 | 2.61 | 1.35 | 151 |
| **RSD [%]** | 2.9 | 2.7 | 4.1 | 3.9 | 13.1 |

In this case the mean MMD of the type MS-3 aerosol generator is 3.3 µm compared to 4.1 µm of the type MS-4 aerosol generator.

The aerosol generator 18 may further have a frame 68 for supporting the aerosol generator 18. The frame 68 may be made from resin and may be provided with flexible engagement members 70 (see figure 2) for releasably fastening the aerosol generator 80 to the body 12. For this purpose, the body 12 has engagement openings 72 for receiving the engagement members 70. The engagement members 70 may be flexible hooks hooking behind the wall 74 of the body 12 defining the engagement openings 72. Thus, the aerosol generator 18 may be released from the body 12 for cleaning purposes.

When the aerosol generator 18 is attached to the body 12, the membrane 61 or the support 80 is pressed against the sealing lip 44 when the aerosol generator 18 is mounted to the body 12. As a result, the membrane 61 or the support 80 seals the reservoir 24 or the body 12 at its lower portion, more particularly at the supply opening 42. The membrane 61 has the liquid side 64. In this regard, the liquid side 64 is facing the supply opening 42 so that the liquid solution in the reservoir 24 is applied to the active area 62 on the liquid side 64 of the membrane 61. When vibrating the membrane 61, the liquid solution passes through the apertures 110 and an aerosol is formed on a side opposite to the liquid side 64. This side is referred to as aerosol side 66 opposite to the liquid side 64.

Moreover, the nebulizer 10 comprises a mixing chamber 20. The mixing chamber has an inlet 76. The inlet 76 is positioned at the inlet side of the mixing chamber 20 facing the aerosol generator 18 and the body 12. The inlet 76 is hence provided at the aerosol side 66 of the membrane 60.

Furthermore, the elastic valve member 19 is provided between the inlet 76 of the mixing chamber 20 and the body 12, for sealing the interface between the mixing chamber 20 and the body 12. Additionally, the elastic valve member 19 has two one-way valves 168, particularly flap valves (inhalation valves), controlling/allowing the flow of external air on diametrical opposite sides into the mixing chamber during inhalation only as described in EP 1 927 373 A1. In other words, the one-way valves 168 are provided to control the supplied air, which allows ambient air to flow into the mixing chamber during the inhalation phases but which, however, prevents exhaled air from entering the mixing chamber and reversely passing the aerosol generator 18 in exhalation phases. The one-way valves 168 therefore allow the generation of a bolus in the mixing chamber 20 reducing the amount of exhaled air flowing into the mixing chamber 20 (which rather flows out via the one-way valve 98 (exhalation valve) of the mouthpiece 21 described later) and preventing aerosol from being flown out of the mixing chamber 20 by the exhaled air. The valves 168 further allow transporting of the aerosol bolus out of the mixing chamber 20 in exhalation phases with potentially remaining exhaled air in the mixing chamber 20 and ambient air. As aerosol has accumulated in the mixing chamber 20 during an exhalation phase, there is available to the patient an increased amount of aerosol, the so-called aerosol bolus, especially at the beginning of an inhalation phase.

The body 12 further has a lug 100 having an opening 102. The mixing chamber 20 has at its upper surface a corresponding protrusion 104. Further, the mixing chamber 20 has at its lower portion cylindrical axes 108 engaged with grooves 106 of the body 12. Thus, the mixing chamber 20 may be rotated about the cylindrical axes 108 upon disengaging the opening 102 of the lug 100 from the protrusion 104 for giving access to the elastic valve member 19 and the aerosol generator for cleaning purposes.

A plenum chamber 23 (see figure 3) is defined between the inlet 76 of the mixing chamber 20, the mixing chamber 20, the mouthpiece 22 (alternatively a mask) and the outlet 94 of the mouthpiece 22 (alternatively the mask). The plenum chamber 23 has a volume of at least 60 cubic centimeters and a volume of not more than 90 cubic centimeters. In the present embodiment, the plenum chamber 23 has a volume of 65 cubic centimeters. The plenum chamber 23 has a length between 104mm and 154mm and/or a width of 40mm and 60mm and/or a height of 30mm and 50mm. The mixing chamber 20 as such has a length L between 54mm and 94mm and/or a width W of 40mm and 60mm and/or a height H of 30mm and 50mm.

The mixing chamber 20 further comprises an interface 78 for attaching a mouthpiece 22. The interface 78 may be a cylindrical socket 88. The mixing chamber 20 further has an outlet 90 at an outlet side of the mixing chamber 20. The outlet 90 is provided at an end opposite to the end at which the inlet 76 is provided. The outlet 90 may be defined by an opening 89 of the cylindrical socket 88.

The mouthpiece 22 has an inlet 92 at one end and an outlet 94 at the opposite end. The inlet 92 is configured to be attached to the interface 78, particularly the socket 88, of the mixing chamber 20. The outlet 94 is configured for being accommodated in the mouth of a user. Additionally, the mouthpiece 22 comprises an exhalation opening 96 closed by a one-way valve 98. The one-way valve 98 is a flap valve releasing the exhalation opening 96 during exhalation of a user so that air may escape through the exhalation opening 96 during exhalation. While inhaling, the one-way valve 98 closes the exhalation opening 96 so that no air is sucked in through the exhalation opening 96 during inhalation.

In Table 5 all data from breath simulation experiments are summarized.

**Tab. 5: Overview of mean data from breath simulation experiments with different modified eFlow nebulizers (Data given for amount of rhGM-CSF)**

| **Aerosol generator type** | | **MS-3** | | **MS-4** | |
|---|---|---|---|---|---|
| | | **Mean** | **SD n=6** | **Mean** | **SD n=6** |
| Delivered Dose DD | µg | 394.95 | 20.29 | 250.65 | 30.39 |
| Delivered Dose DD | % | 60.4 | 3.4 | 38.9 | 4.6 |
| Residue in | % | 14.7 | 4.1 | 37.2 | 6.7 |
| Aerosol Losses (calculated) | % | 25.0 | 2.5 | 23.9 | 2.7 |
| Respirable Dose <5µm | µg (API) | 302.70 | 22.06 | 160.58 | 27.50 |
| Respirable Dose | % | 46.3 | 3.8 | 24.9 | 4.3 |
| Respirable Dose <3.3µm | µg (API) | 182.82 | 18.99 | 87.59 | 15.34 |
| Respirable Dose | % | 28.0 | 3.1 | 13.6 | 2.4 |

With the type MS-3 aerosol generator a delivered dose (DD) of 60% was reached. Calculated with the data determined by laser diffraction this results in a respirable dose < 5 µm (=dose theoretically reaching the lungs) of 46% of the filled rhGM-CSF. The delivered dose of the type MS-4 aerosol generator was only 39%. As a result, the respirable dose < 5 µm was only 25%. This can be explained with the untypically high amount of formulation in the mixing chamber of the aerosol generator. The residue in the type MS-4 aerosol generator was with 37% considerably increased compared to the type MS-3 aerosol generator (15%). This can be explained by a high amount of impacted aerosol in the mixing chamber. Due to the high output rate (TOR) of the aerosol of 1123 mg/min with the type MS-4 aerosol generator and the reduced surface tension of the formulation, the droplets tend to agglomerate and deposit in the mixing chamber. This effect can be reduced by using an even bigger mixing chamber. As can be seen in Table 6 this was tested in a small experiment, were the residue in the nebulizer was determined by weighing after nebulization with a type MS-4-XL nebulizer, that is the type MS-4 aerosol generator combined with a plenum chamber having a volume of more than 90 ml (XL mixing chamber). The residues of the type MS-4-XL aerosol generator were only slightly higher than those of the type MS-3 aerosol generator.

**Tab. 6: Weighted residues after nebulization of 2.4 mL of rhGM-CSF formulation using a modified eFlow MS-3, modified eFlow MS-4 and modified eFlow MS-4-XL**

| Nebulizer | eFlow MS-3 | eFlow MS-4 | eFlow MS-4-XL (n=2) |
|---|---|---|---|
| Residue in mixing chamber | 0.32 mg | 0.86 mg | 0.49 mg |

### References List

10 nebulizer
12 body
14 lid
16 sealing
18 aerosol generator
19 elastic valve member
20 mixing chamber
22 mouthpiece
23 plenum chamber
24 reservoir
26 liquid solution
27 head space
28 circumferential rim
30 opening
32 outer circumferential surface
33 helical path
34 slot
36 protrusion of the lid
38 top surface of the circumferential rim
40 counter surface
42 supply opening
44 sealing lip
46 contact opening
48 vent
50 notch
52 inner circumferential surface
54 bottom
56 rib
58 top surface of the rib
60 vibratable membrane
62 active area
64 liquid side
66 aerosol side
68 frame
70 engagement member
72 engagement opening
74 wall
76 inlet of the mixing chamber
78 interface
80 support
82 piezoelectric actuator
84 contact
86 controller
88 socket
90 outlet of the mixing chamber
92 inlet of the mouthpiece
94 outlet of the mouthpiece
96 exhalation opening
98 one-way valve
100 lug
102 opening
104 protrusion of the mixing chamber
106 groove
108 axis
110 aperture
112 nozzle portion
114 first portion
116 second portion
118 recess
120 opening of the recess
122 bottom of the recess
124 circumferential side wall of the recess
126 single layer
128 entrance opening
130 exit opening
162 sealing ring
164 sealing hinge
166 center sealing
168 one-way valve of the elastic valve member
API Active pharmaceutical ingredient
CU Control Unit (device for the electronic control of the nebulization)
DD Delivered Dose; Amount of drug delivered on inhalation filter [mg or % of filled drug amount]
GM-CSF granulocyte-macrophage colony stimulating factor GSD Geometric Standard Deviation = Width of deviation curve of droplet sizes
MMD Mass Median Diameter is the droplet diameter that divides the frequency distribution in half; fifty percent of the aerosol mass has droplets with a larger diameter, and fifty percent of the aerosol mass has droplets with a smaller diameter
Neb time Nebulization time [min]
Residue Residual amount in nebulizer [mg]
RF< x µm Respirable Fraction < x µm is the percentage portion of the droplet size less than a diameter of x µm SD Standard deviation
SDS sodium dodecyl sulphate
rhGM-CSF recombinant human granulocyte-macrophage colony stimulating factor
RSD Relative standard deviation
TOR Total output rate [mg/min] = Mass of aerosol delivered per minute
TV Tidal volume

## Claims

1. A drug-device combination comprising:
a liquid solution (26) containing a biologic; and
a nebulizer (10) for aerosolization of the liquid solution (26), the nebulizer (10) comprising:
a reservoir (24) for holding the liquid solution (26), the reservoir (24) having a vent (48) for maintaining atmospheric pressure in the reservoir (24);
a vibratable membrane (60) having an active area (62) with apertures (110), wherein the liquid solution (26) is feedable to the active area (62) at a liquid side (64) of the membrane (60);
a piezoelectric actuator for vibrating the membrane (60), whereby an aerosol of the liquid solution (26) is generated at the active area (62) at an aerosol side (66) of the membrane (60), the aerosol side (66) of the membrane (60) being opposite to the liquid side (64) of the membrane (60);
a plenum chamber (23) having an inlet (76) at an inlet side at the aerosol side (66) of the membrane (60) and an outlet (94) at an outlet side for administering the aerosol, the outlet (94)of the plenum chamber being opposite to the inlet (76) of the plenum chamber (23).

2. The drug-device combination of claim 1, wherein the vent (48) is a passive vent.

3. The drug-device combination of claim 1 or 2, wherein the reservoir (24) is a cup reservoir defined by
a body (12) sealed at a lower portion by the vibratable membrane (60);
the body (12) having a circumferential rim (28) defining an opening (30) for receiving the liquid solution (26) and a sealing surface (38);
a lid (14) for engaging the circumferential rim (28) to close the reservoir (24) and sealing against the sealing surface (38) ;
the vent (48) connecting a head space (27) of the reservoir (24) to atmosphere and being spaced away from the sealed lower portion.

4. The drug-device combination of claim 3, wherein the vent (48) is a notch (50) formed from the sealing surface (38) into the circumferential rim (28) and traversing the circumferential rim (28) connecting the interior of the reservoir (24) and atmosphere.

5. The drug-device combination of anyone of the preceding claims, wherein the plenum chamber (23) has a volume of at least 60 cubic centimeters.

6. The drug-device combination of anyone of the preceding claims, wherein the plenum chamber (23) has a volume of not more than 90 cubic centimeters.

7. The drug-device combination of anyone of the preceding claims, wherein the plenum chamber (23) has a length between 109mm and 154mm, and/or a width of 40mm and 60mm and/or a height of 30mm and 50mm.

8. The drug-device combination of anyone of the preceding claims, wherein the apertures (110) have a size at the aerosol side (66) between 1,5 µm to 5 µm.

9. The drug-device combination of anyone of the preceding claims, wherein the vibratable membrane (60) comprises 500 to 6000 apertures (110).

10. The drug-device combination of anyone of the preceding claims, wherein the apertures (100) are laser drilled and/or laser milled.

11. The drug-device combination of anyone of the preceding claims, wherein the apertures (110) have a nozzle portion (112) at the aerosol side of the active area of the membrane (60), the nozzle portion (112) having a length of less than 20µm.

12. The drug-device combination of anyone of the preceding claims, wherein the liquid solution (26) has a viscosity of 0.900 to 5.000 mPa•s, preferred from 0.950 to 3.500 mPa•s, more preferred from 1.000 to 2.000 mPa•s.

13. The drug-device combination of anyone of the preceding claims, wherein the liquid solution (26) has a surface tension of of 32 mN/m to 108mN/m or preferred of 37 mN/m to 73 mN/m.

14. The drug-device combination of anyone of the preceding claims, wherein the liquid solution (26) has an osmolality of 200 mOsm/kg to 700 mOsm/kg, preferred 200 mOsm/kg to 500 mOsm/kg.

15. The drug-device combination of anyone of the preceding claims, wherein the amount of the liquid solution (26) of one dose is between 1ml and 2ml.

16. The drug-device combination of anyone of the preceding claims, wherein the volume of the reservoir (24) is between 1 ml and 5ml, preferably between 3ml and 5ml.

17. The drug-device combination of anyone of the preceding claims, wherein the biologic comprises one or more proteins and preferred two or more proteins.

18. The drug-device combination of claim 17, wherein one of the proteins is granulocyte-macrophage colony stimulating factor (GM-CSF) and preferred is recombinant human granulocyte-macrophage colony stimulating factor (rhGM-CSF).
